# EUROPEAN PATENT SPECIFICATION

(11) **EP 0 354 492 B1**
(45) Date of publication and mention of the grant of the patent: **31.08.1994**
(21) Application number: 89114467.7
(22) Date of filing: 04.08.1989
(51) Int. Cl.: C07G 17/00, A61K 35/12

(54) **Preparing process of physiologically active substance with anticancer effect and substance obtained thereby**
Verfahren zur Herstellung einer physiologisch aktiven Substanz mit Antikrebs-Aktivität und so erhaltene Substanz
Procédé de préparation d'une substance physiologiquement active ayant une activité anticancéreuse et substance ainsi obtenue

(30) Priority: 10.08.1988 JP 199688/88
(43) Date of publication of application: 14.02.1990
(73) Proprietor: BIO DEFENCE INSTITUTE CO., LTD., Yokohama-shi, Kanagawa (JP)
(72) Inventor: Sato, Ichiei, Takasaki-shi Gunma (JP)
(74) Representative: Leyh, Hans, Dr.-Ing.

(56) References cited:
- EP-A- 0 086 475
- EP-A- 0 090 892
- US-A- 4 261 976
- PATENT ABSTRACTS OF JAPAN, vol. 11, no. 242 (C-438)(2689), August 7, 1987; & JP-A-62 48 631 (DAI ICHI SEIYAKU CO. LTD) 03-03-1987
- PATENT ABSTRACTS OF JAPAN, vol. 9, no. 196 (C-297)(1919), August 13, 1985; & JP-A-60 67 429 (SENDAI BISEIBUTSU KENKYOSHO) 17-04-1985
- PATENT ABSTRACTS OF JAPAN, vol. 8, no. 187 (C-240)(1624), August 28, 1984; & JP-A-59 82 315 (YAMANOUCHI SEIYAKU K.K.) 12-05-1984

## Description

### BACKGROUND OF THE INVENTION

### Field of the Invention

This invention relates to a physiologically active composition with anticancer effect and to a process of preparing the composition.

### Related Art Statement

Japanese Patent Laid-Open No. 57-140726 discloses a refining process of physiologically active substance with anticancer effect.

It is summarized as follows. A physiologically active proteinic substance with anticancer effect is induced by administration of one kind or more than two kinds of a substance with reticuloendothelial activating effect to a mammal, followed by the injection of an endotoxin of gram negative bacteria origin. The crude solution of the physiologically active substance thus-obtained (or that of physiologically active proteinic substance with anticancer effect induced by adding an endotoxin of gram negative bacteria to tissue culture medium containing activated mammalian macrophages) is adsorbed by basic ion exchanger resin, the eluted solution is subjected to gel filtration to collect the substance of 30 K to 70 K molecular weight, and, if necessary, is further treated by affinity chromatography employing Sieber chromic blue-F3G-A to obtain the fraction containing the above mentioned physiologically active proteinic substance, and thus, the refined physiologically active proteinic substance with anticancer effect is obtained.

There are two categories of anticancer agents for the treatment of cancer. One is the so-called "chemotherapeutic agent" which annihilates cancer cells of cancer bearing organism by direct contact, and the other is the so-called "immune-adopting therapeutic agent" which can reinforce and recover permanence or biophylaxis mechanism of cancer bearing organism weakened due to proliferation of cancer cells (or tissue).

The chemotherapeutic agent may cause adverse side effects such as injuring the normal cells with its cytotoxic effect intended for cancer cells, and its dose is constrained to lower doses and this may cause ineffectiveness against cancer cells depending upon their location, or they may gain resistance.

To cope with such problems, attempts have been made to transfer the anticancer agent to the portion of primary site with high concentration therein. For example, a monoclonal antibody-conjugated anticancer agent, which can attack the portion of cancer, has been tried.

On the other hand, the immune therapeutic agent is used for the purposes of obtaining antitumor effect by cytokine such as lymphokine or monokine induced by activating the immune system which has been inactivated or inhibited with the bearing of cancer.

The immune therapeutic agent, therefore, rarely causes adverse effects on normal cells during long term administration, and as it does not directly attack the cancerous cells (or tissue) it does not exert an immediate effect.

The present inventor studied extensively the immune activating effect of a physiologically active composition produced in ascites of a mammal following transplanting of cancer cells, and established a technique to produce a physiologically active composition with anticancer effects from the ascites of a cancer bearing organism.

The present invention provides a physiologically active composition with anticancer effects and the method of preparation thereof.

### SUMMARY OF THE INVENTION

The process of the present invention is started by transplanting cancer cells in an abdominal cavity of a mammal, followed by administration of an anticancer agent in the same abdominal cavity, and further followed by administration of spleen cells extracted from a normal mammal into the vein of the mammal, collecting ascites retained in the cavity after the elapse of a specific time period, fractionating its supernatant liquid by Cohn's fractionation, and extracting the physiologically active composition with anticancer effect from the 5th fraction.

The invention also provides the extraction of the physiologically active composition with anticancer effect by extraction of the V fraction of Cohn's fraction from supernatants of ascites obtained from the abdominal cavity of a cancer patient who has been improved by immune surveillance therapy.

Measured by verifying cancer chemotherapy requirement with significant life elongation, it was indicated that in cancer bearing organisms pre-administered with an anticancer agent and followed by a dose of fraction containing the biologically active composition obtained by the present invention, an apparent life span elongation as compared to the unadministered cancer bearing organisms was achieved. This result revealed the presence of a physiologically active composition with significant anticancer effect in the above mentioned fraction.

Thus, according to the invention, a very useful composition with anticancer effect is obtained.

Hereunder are examples of practical application which explain the invention in detail, although the invention is not limited to those examples.

### BRIEF EXPLANATION OF DRAWINGS

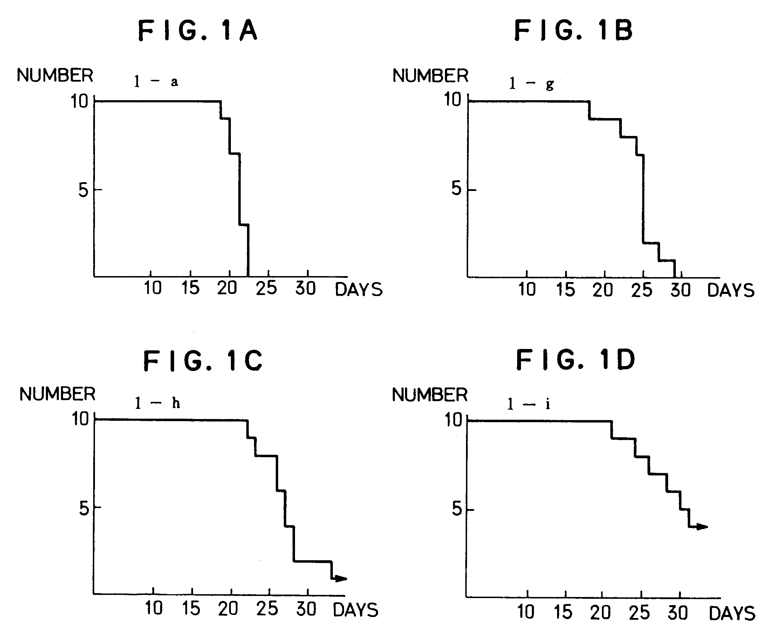

Figs. 1A to 3D are survival life curves of physiologically active compositions of the present invention and curves of control groups, illustrating the antitumor effects of the biologicaly active composition according to the present invention.

### DETAILED DESCRIPTION OF EXAMPLES

### Example 1

### 1. Preparation of Ascites containing Cancer Cells

For proliferation, MM46 cells, a lineage of a mouse ascites mammary cancer cell, were transplanted into the abdominal cavity of C3H/He mice (8 week old, male) at the cell number of 1 X 10⁵ to 10⁶ /mouse. Ten days later, the ascites retained in the mouse abdominal cavities were collected, centrifuged (800 rmp for 10 minutes) to isolate the MM46 cells. Then, MM46 cell pellets were suspended in physiological saline solution to remove red blood cells by conducting centrifugation (800 rpm for 10 minutes) two or three times. The cell pellets were resuspended in physiological saline solution at a cell density of 2 X 10⁵ cells/ml.

The above manipulation was conducted under sterile and ice-water cooling conditions.

### 2. Preparation of Splenic Cells

BALB/c mouse spleen cells were prepared by usual manner, i.e. the spleens isolated from normal BALB/c mice (8 week old, male) under sterile conditions were immediately crashed and immersed in Hank's solution, then filtered through stainless mesh (#200) in 60 mm scale Petri dish to obtain a solution of free cells which was then, washed with Hank's solution subjected to centrifugation.

Next, a mixed solution of 0.83 % NH₄Cl : 0.17 mol Tris-HCl = 9 : 1 (pH = 7.65) was sterilized by filtration. To the spleen cell pellets, 10 times of said buffer solution was added. After quick agitation, this mixture was centrifuged (1,000 rpm for 10 minutes) under low temperature, and, then, the residual red blood cell pigment was removed by washing 3 times with physiological saline water to obtain a spleen cell preparation, which was used as suspension in physiological saline solution at 4°C for 16 hours.

### 3. Preparation of the physiologically active composition

MM46 cells of process 1 were transplanted to abdominal cavities (2 X 10⁴ cells per mouse) of C3H/He mice (8 week old mouse), at day 3 after which, "Aclarcinon" (Aclarbicin hydrochloride manufactured by Yamanouchi Pharmaceutical Co., here-in-after referred to as "ACR") was administered in the abdominal cavities (3 mg/kg) as an anticancer agent. At day 4 to day 6 after the transplantation, spleen cells of BALB/c mouse prepared according to the above process 2 were administered in to the tail vain of the above mentioned C3H/He mice (1 X 10⁶ cells per mouse). At day 21 to 25 after the transplantation, ascites retained in the abdominal cavities of the C3H/He mice were collected (ascites containing blood was not used for next experiments). In the next step, cell residues such as MM46 cells and mouse blood cells contained in the above mentioned ascites were removed by centrifugation (10,000 rpm for 20 minutes), the supernatant liquid was freeze dried and condensed which was subjected to Cohn's fractionation (organic solvent fractionation) to obtain the V fraction.

### Antitumor Test

The following experiments were conducted to examine the antitumor effect of the V fraction of Cohn's fractionation obtained under the above mentioned process (here-in-after referred to as "ascites derivatives").

### Test methods:

Three kinds of ascites derivatives were prepared under the above mentioned process, and 2 kinds of ascites derivatives were prepared as control (Table 1).

The ascites derivative marked Sample No. 1 in Table 1 is the control obtained by treating according to the above mentioned process 3 the ascites which had been extracted from the abdominal cavities of C3H/He mice on the 21st day after transplantation of the MM46 cells, ACR and spleen cells not being administered.

By the way, mice to which only MM46 cells were transplanted retained ascites in the abdominal cavities, and most of them died on the 18th or 19th day or around (mostly from serum ascites), so that few mice were left to extract ascites from.

**Table 1**

| PREPARATION OF ASCITES DERIVATIVES | | | | |
|---|---|---|---|---|
| Sample No. | Ascites extraction day | Ascites collection from mouse treated with | Number of mice used for ascites collection | Yield (mg) |
| 1 | day 21 | MM46 cells only | 4 | 879 |
| 2 | day 21 | MM46 cells + ACR (3 days later) | 9 | 1671 |
| 3 | day 21 | MM46 cells + ACR (3 days later) + spleen cells (4 days later) | 11 | 1796 |
| 4 | day 21 | MM46 cells + ACR (3 days later) + spleen cells (6 days later) | 8 | 1620 |
| 5 | day 21 | MM46 cells + ACR (3 days later) + spleen cells (6 days later) | 4 | 522 |

The ascites derivative marked Sample No. 2 is the control extracted by treating according to the above mentioned process 3 the ascites extracted from the abdominal cavities of C3H/He mice on the 21st day after the transplantation of MM46 cells, while ACR being administered (3 mg/kg) to the mice 3 days after the transplantation.

Samples Nos. 3, 4 and 5 are ascites derivatives fractionated according to the above mentioned Example while each sample was prepared applying various time periods between the transplantation of MM46 cell and the administration of the spleen cells (4 to 6 days) or the collection of ascites (21 to 25 days).

Ascites derived Sample Nos. 3, 4 and 5 are yellowish in color while the turbidity usually present in MM46 cell rich solutions was less present than in Sample Nos. 1 and 2 (in case that ascites collected from some nice contained a little hemoglobin, it was not used for Cohn's fractionation).

In the next step, MM46 cells prepared according to the the abovementhioned process 1 were transplanted (2 X 10⁴ cells per mouse) into the abdominal cavities of C3H/He mice (8 week old, male) of the test groups (10 mice per group) respectively, and, then, ACR (10 mg/kg) was administered to respective abdominal cavities on the next day.

Further, on the following day, ascites derivative Sample Nos. 1 to 5 were respectively administered (12.5 mg/kg) for 10 days continuously to observe casualties in the following 45 days, the results being described in Table 2 (1-i, 1-j, 1-k).

The same procedure was carried out as a control to those mice to which ACR was not administered, or to those mice administered with commercially sold albumin (Sigma Co., Ltd. Lot No. 45F-9339) (12.5 mg/kg) instead of mouse ascites derivatives after ACR administration.

The test results were assessed according to the efficacy assessment standards of cancer chemotherapy to determine the median survival time (here-in-after referred to as "M.S.T.") or the average survival days ratio (T/C %) and the average survival days and the survival days of the median mouse (the 5th died mouse).

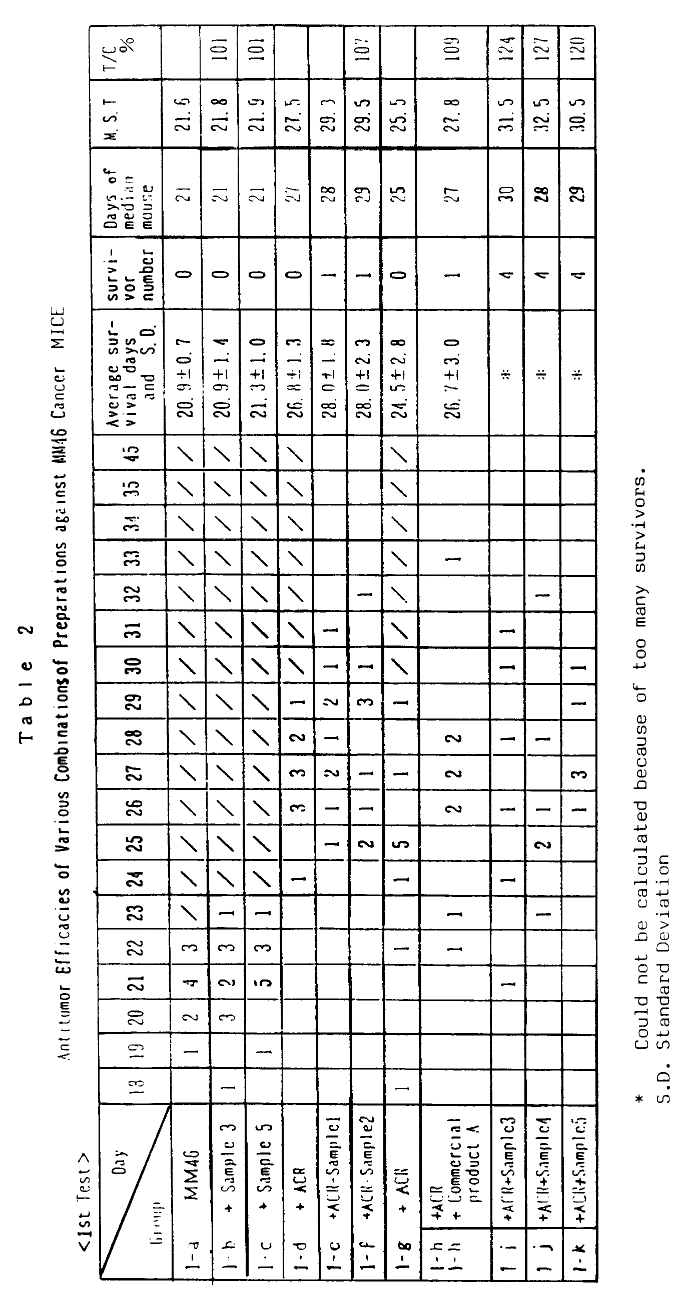

### Test Results (Table 2)

Of the control group (1-a) mice, to which only MM46 cells were transplanted, from about day 10 ascites were found rapidly to retain in abdominal cavities while mice began to die around day 19 following transplantation, and all mice had died at day 22.

The average survival days of mice for the control groups (1-b or 1-c), to which ascites derivatives (Sample 3 or 5) were administered continuously for 10 days after transplantation of MM46 cells without ACR administration, were not substantially different from those of the above mentioned untreated group or around day 21, no significant antitumor effect was observed in these cases.

Namely, no life elongation effect was demonstrated with ascites derivatives of the present Example alone in mice bearing MM46 cells.

Mice of the control groups (1-d and 1-g), to which MM46 cells were transplanted and ACR was administered on the following day but ascites derivatives were not administered, survived by several days longer than that of the above mentioned control groups (1-b and 1-c), to which ascites derivatives were administered but no ACR was administered, and all of the mice of these groups (1-d and 1-g) had died on day 29.

Of control group (1-e), to which ascites derivative Sample No. 1 was administered for 10 days continually following transplantation of MM46 cells and ACR on the following day, control group (1-f), to which ascites derivatives Sample No. 2 was administered for 10 days continually, and control group (1-h) to which commercially sold human serum albumin was administered for 10 days continually, one mouse in each group survived. These results showed no life elongation effect in each case for Sample No. 1.

The mice belonging to groups (1-i, 1-j, 1-k), to which ACR and the ascites derivatives (Sample No. 3, 4 and 5) were administered, survived for days with high survival rate beyond 45 days. No significant differences in life elongation period were caused among Sample Nos. 3, 4 and 5.

The survival life curves for the above tests of typical groups are shown in Figs. 1A to 1D.

In the next, Samples No. 3'₁ and No. 3'₂ ascites derivatives were prepared in the same manner as Sample No. 3, with which Tests No. 2 and No. 3 were conducted in the same manner as Test No. 1, results of which are shown in Table 3.

In these tests, the group to which physiological saline solution was administered for 10 days continually in the same dose (0.1 ml each time per mouse) as the test Samples after transplantation of MM46 cells, the group to which ACR was administered after transplanting MM46 cells, and the group to which commercially sold human serum albumin was administered instead of ascites derivatives for 10 days continually were chosen as the control groups.

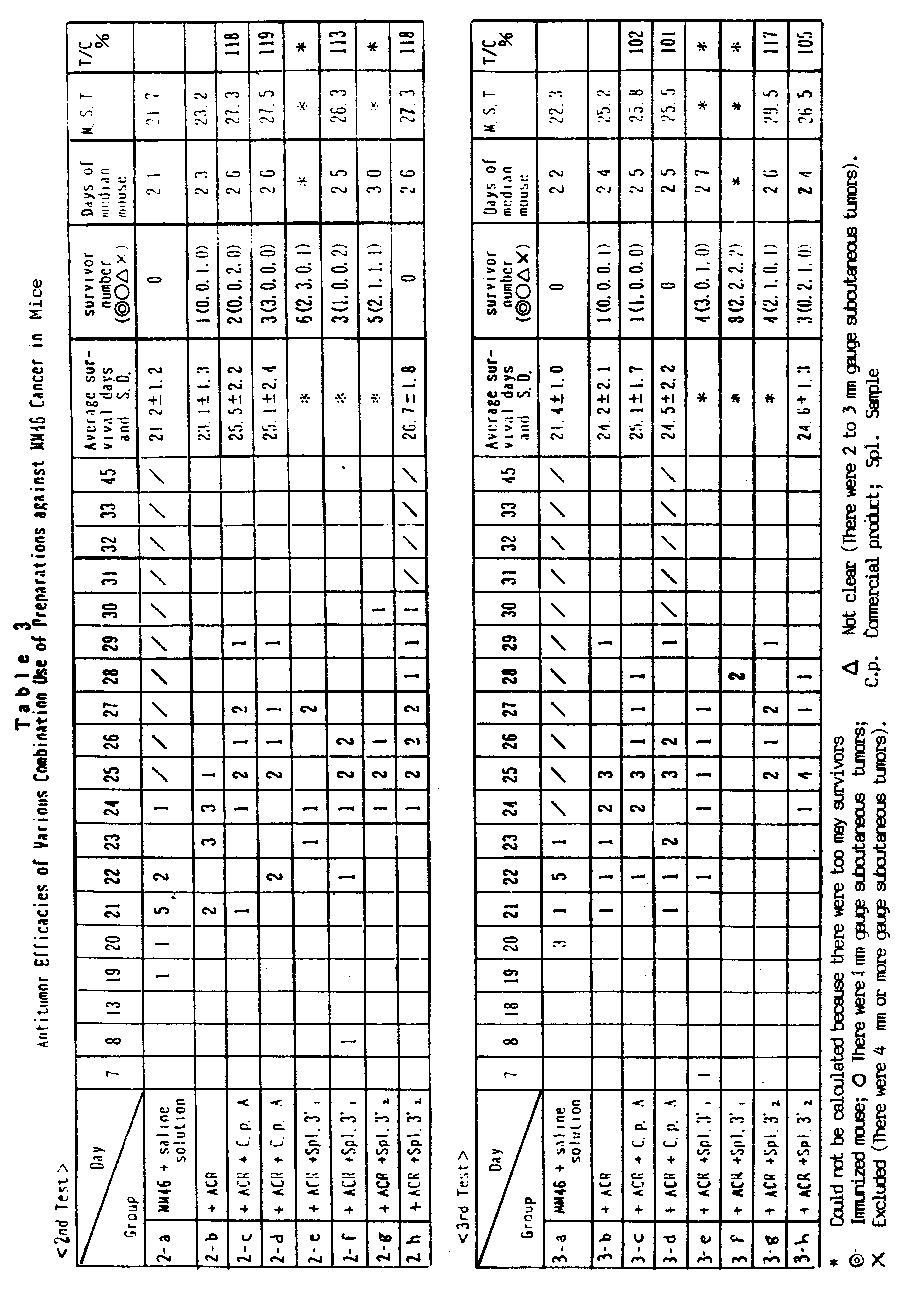

### Test Results (Table 3)

Control groups (2-b and 3-b), to which ACR was administered following transplantation of MM46 cells, showed some more life elongation effects than control groups (2-a and 3-a) to which only physiological saline solution was administered, but ultimately only one mouse survived in each group (2-b and 3-b). Moreover, control groups (2-a and 3-a) showed no life elongation effect by stab stimulus of injection needle.

Control groups (2-c and 3-c) to which ACR and commercially sold human serum albumin was administered after transplantation of MM46 cells, shored some more life elongation effects than control groups (2-b and 3-b), but only one mouse or two mice survived at the end.

On the other hand, groups (2-d, 2-e, 3-d and 3-e) to which ACR and ascites derivatives (Sample Nos. 3'₁ or 3'₂) prepared according to the above mentioned process were administered showed later perishing dates than the control groups mentioned above, while the number of survivors at day 45 after the transplantation was increased, thus, exerting very significant life elongation effects.

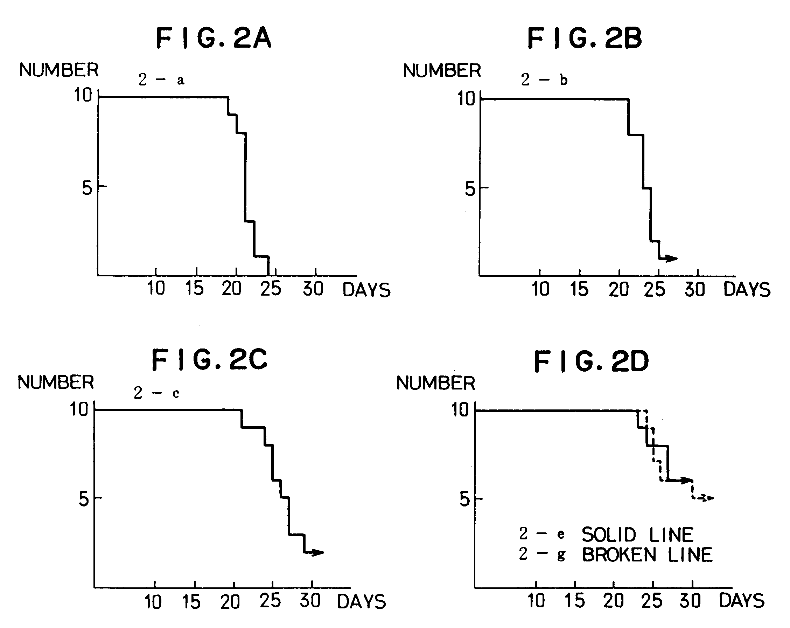

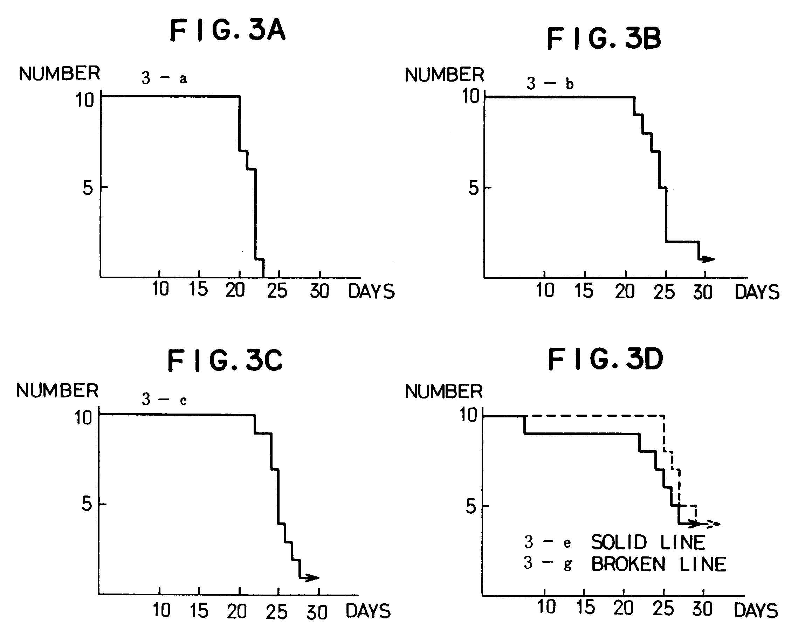

The life elongation curves resulting from the 2nd and 3rd tests mentioned above of typical groups are shown in the Figs. 2 and 3.

From these test results, it becomes evident that the physiologically active composition with prominent anticancer effect is produced in the ascites derivatives obtained by the process of the present Example.

### Example 2

Immune therapy surveillance (the so-called "Sato's Therapy" as described in "Research Report, 1979: Research on Cancer Immune Surveillance Therapy" published by Gan Men-eki Shinkoh Zaidan (Cancer Immunity Promotion Foundation), Japan) was applied to cancer patients, and ascites were extracted from abdominal cavities of patients showing signs of improvement. Then, cellular residues were removed from ascites by centrifugation as has been done in the above-mentioned Example 1 process, while its supernatants were freeze-dried, and then Cohn-fractionation was conducted to obtain its Cohn's V fraction.

The following Cases I to III show the specific antitumor effect of the physiologically active composition (BRP) comprised in this Cohn V fraction.

### [Case I]

Sex: female
Age: 44
Decease: A.T.L.A.
Clinical findings and treatment:
(1) Consulted on fever and systematic exhaustion, and diagnosed as A.T.L.A.
(2) Hospitalized and examined.
(3) Injected Aclarcinon 10 mg, Pepleo 10 mg, MMC 2 mg, Oncobin 1 mg, Predonin 20 mg on (i) day 2, (ii) day 5, and (iii) day 9 after examination.
(4) On the 11th day, 24 ml destilled water containing 1.25 % of BRP obtained in Example 2 were infused.
(5) The patient showed a normal hemogram and immune response these treatments below, and was dehospitalized on the 77th day after BRP treatment, upon complete recovery.

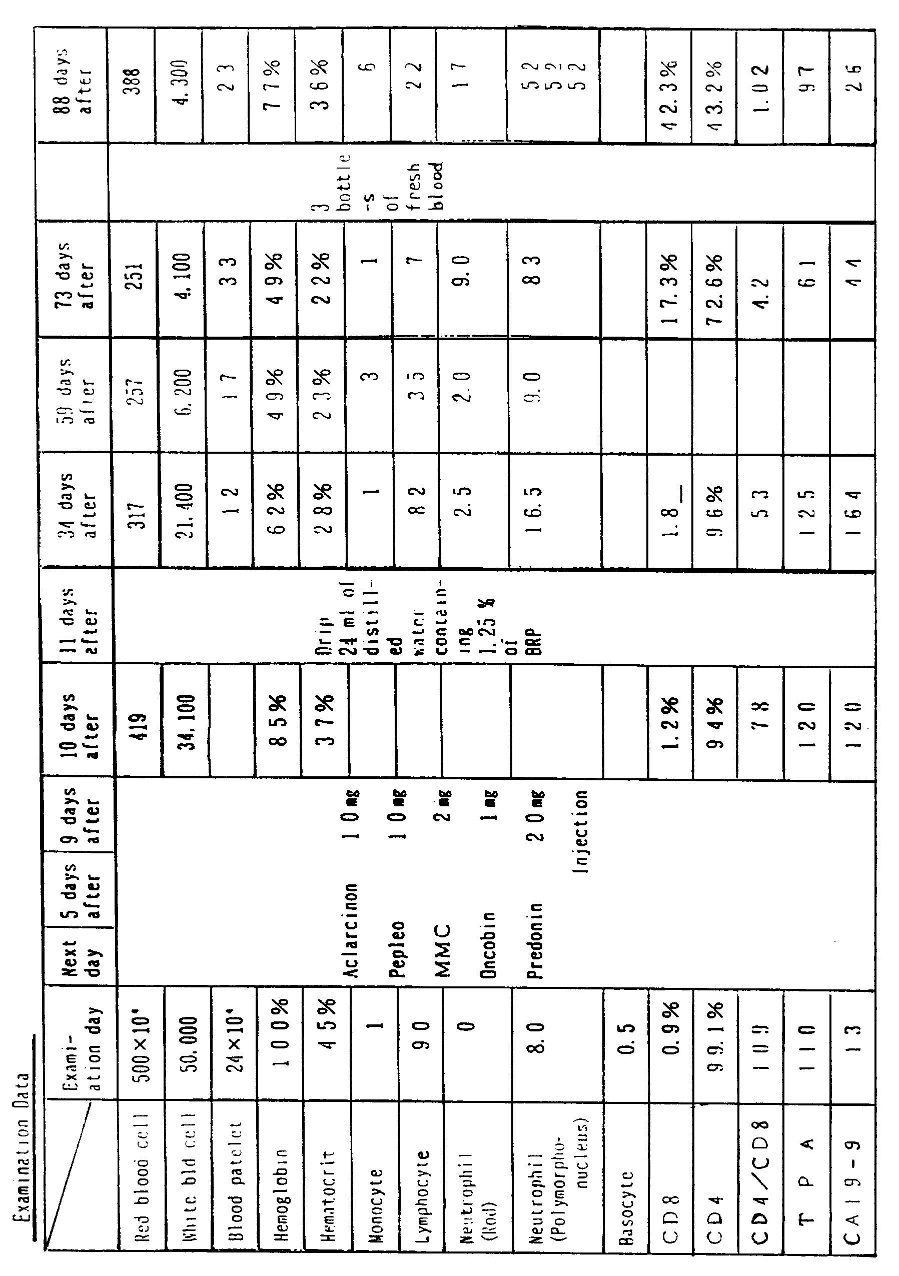

### [Case II]

Sex: female
Age: 38
Disease: metastasis of left breast cancer to lungs.
Anamnesis: After 11 years and 8 months from radical curing operation of left breast cancer, metastasis to both lungs was found which gradually expanded.
Pleural effusion in both lungs were found in the following year.
Later, tumor shadow and hydrothrax were found in lower parts of both lungs.
Treatment:
(1) Hospitalized
(2) 31 days later, 24 ml of distilled water for injection containing 1.25 % of BRP obtained under Example 2 were injected intravenously.
(3) Favorable improvements after intravenous injection with BRP, such as narrowed lung shadow, improved hemogram after 2 weeks, normalized CD4/CD8, reduced tumor marker, have been observed.

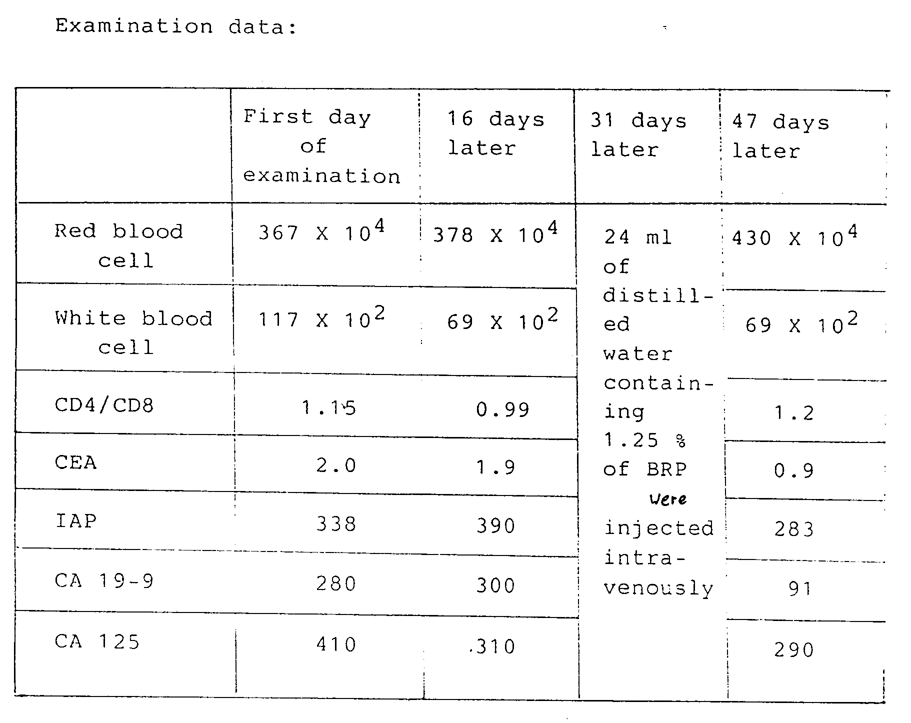

### [Case III]

Sex: male
Age: 47
Dicease:
1. metastasis of upper throat cancer to the lung
2. right cancerous pleurisy
3. metastasis to right thigh and to liver
History and clinical finding when hospitalized:
Upper throat cancer was operated, followed by radioactive therapy.
Two years and two months later, the patient was examined for sputum, and a cavitary shadow on right lung was found. This indicates that metastasis to lung has been confirmed.
Eight months later metastasis nestle was found in right femur by bone scintillation while the chest shadow was expanded.
One month later, the patient appealed of cough and sputum, and of pains from right chest to right shoulder. Chest X-ray photograph revealed a diffused shadow in right chest, while right chest X-ray photograph 2 cm diameter osteolysis figure.
Treatment:
(1) Hospitalized
(2) Twelve days after first examination, 24 ml of distilled water for injection containing 1.25 % of BRP obtained under Example 2 were injected intravenously.
(3) Sixty-two days after the first examination, 24 ml of distilled water for injection containing 1.25 % of BRP obtained under Example 2 were injected into the abdominal cavity.
(4) From the 33rd day after first examination, the chest sting alleviated gradually while difficulty of breathing alleviated, which altogether disappeared on the 117th day.

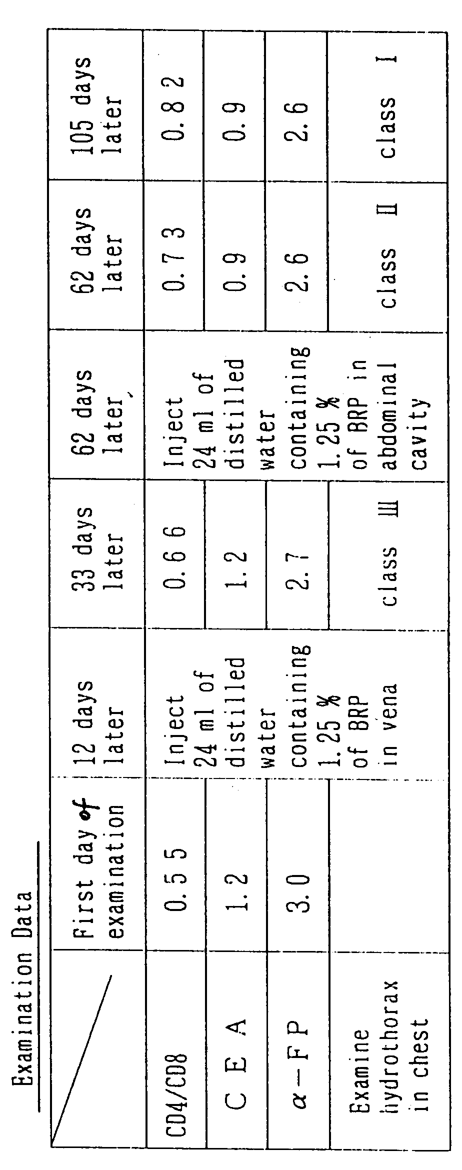

## Claims

1. A process for preparing a physiologically active composition with anticancer effect comprising Cohn's V fraction, the process comprising the following steps:
- transplanting cancerous cells into the abdominal cavity of a mammal;
- administering an anticancer agent to the abdominal cavity;
- intravenously administering spleen cells extracted from a normal mammal of same kind into said mammal, into which cancer cells have been transplanted;
- collecting the ascites retained in said abdominal cavity after elapse of a predetermined time period; and
- fractionating the supernatant liquid of said ascites by Cohn's fractionation to isolate Cohn V fraction of said supernatant liquid.

2. The process according to claim 1, wherein said supernatant of the ascites collected from the abdominal cavity is centrifuged and has been removed from cellular residues before Cohn's fractionation procedure.

3. The process according to claim 1, wherein said mammal is a mouse.

4. The process according to claim 1, wherein said anticancer agent is aclarbicin-hydrochloride.

5. A physiologically active composition having an anticancer effect, which composition has been prepared by the process according to any of the preceding claims 1 to 4.

6. A process for preparing a physiologically active composition with anticancer effect comprising Cohn's V fraction, the process comprising the following steps:
- collecting the ascites from an abdominal cavity of a cancer patient, who has improved by immunosurveillance therapy; and
- fractionating the supernatant liquid of said ascites by Cohn's fractionation to isolate Cohn V fraction of said supernatant liquid.

7. The process according to claim 6, wherein said supernatant liquid of the ascites extracted from said abdominal cavity is centrifuged and has been removed from cellular residues before Cohn's fractionation.

8. A physiologically active composition having an anticancer effect, which composition has been prepared by the process according to claim 6 or 7.

## Patentansprüche

1. Verfahren zur Herstellung einer physiologisch aktiven Zusammensetzung mit einer Antikrebs-Wirkung, die die Cohn-Fraktion V enthält, wobei das Verfahren die folgenden Schritte aufweist:
- Krebszellen in die Bauchhöhle eines Säugetiers zu transplantieren;
- der Bauchhöhle ein Antikrebs-Mittel zu verabreichen;
- Milzzellen, die aus einem normalen Säugetier derselben Art extrahiert worden sind, intravenös an das Säugetier zu verabreichen, in das die Krebszellen transplantiert worden sind;
- das Bauchwasser (Aszites), das nach Ablauf einer bestimmten Zeit in der Bauchhöhle zurückgeblieben ist, zu sammeln; und
- den überstand des Bauchwassers mittels Cohn-Fraktionierung zu fraktionieren und die Cohn-Fraktion V des überstandes zu isolieren.

2. Das Verfahren nach Anspruch 1, wobei der überstand des Bauchwassers, das aus der Bauchhöhle gesammelt worden ist, zentrifugiert wird und vor dem Vorgang der Cohn-Fraktionierung von zellulären Rückständen befreit worden ist.

3. Das Verfahren nach Anspruch 1, wobei das Säugetier eine Maus ist.

4. Das Verfahren nach Anspruch 1, wobei das Antikrebs-Mittel Aclarbicin-Hydrochlorid ist.

5. Physiologisch aktive Zusammensetzung mit einer Antikrebs-Wirkung, die mittels des Verfahrens nach einem der vorangehenden Ansprüche 1 bis 4 hergestellt worden ist.

6. Verfahren zur Herstellung einer physiologisch aktiven Zusammensetzung mit einer Antikrebs-Wirkung, die die Cohn-Fraktion V enthält, wobei das Verfahren die folgenden Schritte umfaßt:
- das Bauchwasser (Aszites) aus einer Bauchhöhle eines Krebspatienten, der sich aufgrund einer Therapie der Immunüberwachung (immunosurveillance therapy) erholt hat, zu sammeln; und
- den überstand des Bauchwassers mittels Cohn-Fraktionierung zu fraktionieren und die Cohn-Fraktion V des überstandes zu isolieren.

7. Das Verfahren nach Anspruch 6, wobei der überstand des Bauchwassers, das aus der Bauchhöhle extrahiert worden ist, zentrifugiert wird und vor dem Vorgang der Cohn-Fraktionierung von zellulären Rückständen befreit worden ist.

8. Physiologisch aktive Zusammensetzung mit einer Antikrebs-Wirkung, die mittels des Verfahrens nach Anspruch 6 oder 7 hergestellt worden ist.

## Revendications

1. Procédé de préparation d'une composition physiologiquement active douée d'activité anticancéreuse comprenant la fraction V de Cohn, ce procédé comprenant les étapes suivantes:
- la transplantation de cellules cancéreuses dans la cavité abdominale d'un mammifère;
- l'administration d'un agent anticancéreux dans la cavité abdominale;
- l'administration par voie intraveineuse audit mammifère, dans lequel on a pris soin de transplanter des cellules cancéreuses, de splénocytes extraites à partir d'un mammifère normal appartenant au même genre;
- le prélèvement des ascites contenues dans ladite cavité abdominale au bout d'un intervalle de temps prédéfini; et
- le fractionnement du surnageant liquide desdites ascites par fractionnement de Cohn afin d'isoler la fraction V de Cohn à partir dudit surnageant liquide.

2. Procédé selon la revendication 1, dans lequel ledit surnageant des ascites prélevées dans la cavité abdominale est centrifugé et débarrassé des débris cellulaires avant de procéder au fractionnement de Cohn.

3. Procédé selon la revendication 1, dans lequel ledit mammifère est une souris.

4. Procédé selon la revendication 1, dans lequel ledit agent anticancéreux est le chlorhydrate d'aclarbicine.

5. Composition physiologiquement active douée d'activité anticancéreuse, laquelle composition ayant été préparée par un procédé selon l'une quelconque des revendications précédentes 1 à 4.

6. Procédé de préparation d'une composition physiologiquement active douée d'activité anticancéreuse comprenant un fractionnement par la technique de Cohn, ce procédé comprenant les étapes suivantes:
- le prélèvement des ascites à partir de la cavité abdominale d'un malade cancéreux, dont l'état s'est amélioré grâce à une thérapie capable de renforcer la surveillance immunitaire; et
- le fractionnement du surnageant liquide desdites ascites par la technique de Cohn afin d'isoler la fraction V de Cohn à partir dudit surnageant liquide.

7. Procédé selon la revendication 6, dans lequel ledit surnageant liquide des ascites extraites de ladite cavité abdominale est centrifugé et débarrassé des débris cellulaires avant de procéder au fractionnement de Cohn.

8. Composition physiologiquement active douée d'activité anticancéreuse, laquelle composition ayant été préparée par le procédé selon la revendication 6 ou 7.
